# EUROPEAN PATENT APPLICATION

(11) **EP 4 696 758 A1**
(43) Date of publication of application: **18.02.2026**
(21) Application number: 24797083.3
(22) Date of filing: 24.04.2024
(51) Int. Cl.: C09J 189/00, C09J 11/06

(54) **ADHESIVE COMPOSITION**

(30) Priority: 27.04.2023 JP 2023073852
(71) Applicant: TEIJIN LIMITED, Kita-ku, Osaka-shi, Osaka 530-0005 (JP)
(72) Inventor: HASHIMOTO, Takuya, Osaka-shi, Osaka 530-0005 (JP)
(74) Representative: Hoffmann Eitle
(86) International application number: PCT/JP2024/016131
(87) International publication number: WO 2024/225337

(57) **Abstract**

Provided is an adhesive composition that includes a first liquid containing gelatin having an amino group; and a second liquid containing a crosslinking agent having at least two functional groups capable of crosslinking reaction with the amino group in one molecule and a polyethylene glycol skeleton, in which a pH of the first liquid at 25°C is from 6.5 to 9.0, a storage modulus of the first liquid at 25°C is 1 Pa or less, an amino group concentration of the first liquid is from 4.0 mM to 40.0 mM, a functional group concentration in terms of the functional group contained in the crosslinking agent in the second liquid is from 2.0 mM to 65.0 mM, and a weight average molecular weight of the crosslinking agent is 10000 or more.

## Description

### Technical Field

The present disclosure relates to an adhesive composition.

### Background Art

Tissue adhesives are used not only for wound closure but also as hemostatic agents to suppress blood leakage from a vascular suture site, as sealants to prevent air leaks from a lung, and as regenerative scaffold materials which are mixed with cells and injected into an affected site, and thus have a wide range of applications. Tissue adhesives are roughly classified into liquid tissue adhesives and sheet tissue adhesives depending on the form of use. The liquid tissue adhesive may be sprayed as an aqueous solution to an application site with a syringe, a spray, or the like at the time of surgery. Liquid tissue adhesives are mainly classified into blood preparations and non-blood preparations.

Fibrin glue, which is a blood preparation, combines fibrin and thrombin, and exerts adhesive strength by a coagulation reaction of both (see, for example, Patent Literature 1).

On the other hand, research on a tissue adhesive using gelatin which is a non-blood preparation as a raw material is actively being conducted. Non-Patent Literature 1 and 2 disclose a tissue adhesive prepared by chemically crosslinking hydrophobized cod-derived gelatin with multibranched N-hydroxysuccinimidized polyethylene glycol (PEG) (hereinafter, referred to as multibranched PEG). It has been reported that these tissue adhesives exhibit a tissue adhesive strength from 1 kPa to 5 kPa (from 0.1 N/cm² to 0.5 N/cm²).
Patent Literature 1: Japanese Patent Application Laid-Open (JP-A) No. 2010-069031
Non-Patent Literature 1: R. Mizuta, T. Taguchi et al., Acta Biomaterialia 121 (2021) 328-338
Non-Patent Literature 2: Y. Mizuno T. Taguchi et al., International Journal of Biological Macromolecules 163 (2020) 2365-2373

### SUMMARY OF INVENTION

### Technical Problem

However, in the invention described in Patent Literature 1, the coagulation reaction between fibrin and thrombin is instantaneously completed, and it is thus extremely difficult to use the invention on a complicated adhesion surface. Therefore, there is a problem that handling properties are insufficient.

In addition, the tissue adhesives described in Non-Patent Literature 1 and 2 using gelatin as a raw material have a problem that the adhesive strength is not sufficient in a case in which application to knee cartilage or the like is assumed.

Under such circumstances, there is a need for a tissue adhesive in which two liquids that promote crosslinking reactions can be filled into respective syringes and injected, in which the solidification time is controlled so as to solidify within an appropriate time, and which has an appropriate adhesive strength.

The disclosure has been made in view of the above conventional circumstances, and an object thereof is to provide an adhesive composition which satisfies fillability into a two-liquid syringe and handling properties for injection, and in which a chemically crosslinked gel produced by mixing two liquids can exhibit favorable adhesive strength within an appropriate time.

In the disclosure, the expression "can exhibit favorable adhesive strength within an appropriate time" means that, gelation can occur within 10 seconds or more and less than 600 seconds by mixing two liquids, in an environment of 25°C.

### Solution to Problem

As a result of intensive studies, the present inventors have found that the above problems can be solved by mixing a first liquid containing gelatin and a second liquid containing a predetermined crosslinking agent, and have completed the present invention.

Specific means for achieving the above object are as follows.
<1> An adhesive composition including:
   a first liquid containing gelatin having an amino group; and
   a second liquid containing a crosslinking agent having at least two functional groups capable of crosslinking reaction with the amino group in one molecule and a polyethylene glycol skeleton, in which:
      a pH of the first liquid at 25°C is from 6.5 to 9.0,
      a storage modulus of the first liquid at 25°C is 1 Pa or less,
      an amino group concentration of the first liquid is from 4.0 mM to 40.0 mM,
      a functional group concentration in terms of the functional group contained in the crosslinking agent in the second liquid is from 2.0 mM to 65.0 mM, and
      a weight average molecular weight of the crosslinking agent is 10000 or more.
<2> The adhesive composition according to <1>, in which the functional groups include an N-hydroxysuccinimidyl (NHS) group.
<3> The adhesive composition according to <1> or <2>, in which the crosslinking agent includes a compound represented by the following Formula (A).

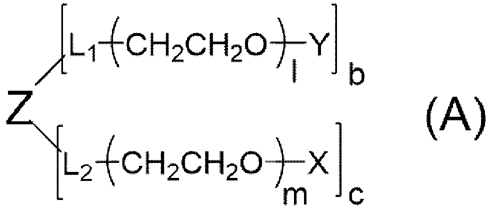

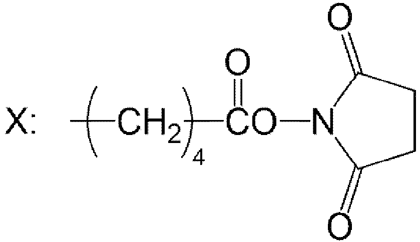

In Formula (A), Z represents a (b+c)-valent organic group, Y represents a hydrogen atom or a monovalent organic group, L₁ and L₂ each independently represent -O- or -O-CONH-(CH₂)₃-O-, b represents an integer from 0 to 5, c represents an integer from 3 to 8, (b+c) represents an integer from 3 to 8, l represents 0 or an integer of 1 or more, and m represents an integer of 1 or more.

<4> The adhesive composition according to any one of <1> to <3>, in which the crosslinking agent includes a compound represented by the following Formula (B).

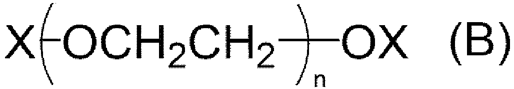

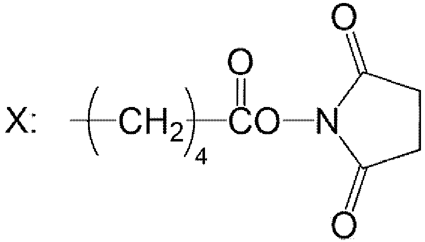

In Formula (B), n represents an integer of 1 or more.

<5> The adhesive composition according to any one of <1> to <4>, in which a pH of the second liquid at 25°C is from 3.0 to 8.0.

<6> The adhesive composition according to any one of <1> to <5>, in which a difference between the pH of the first liquid at 25°C and a pH of the second liquid at 25°C is from 0 to 6.0.

### Advantageous Effects of Invention

According to the disclosure, it is possible to provide an adhesive composition which satisfies fillability into a two-liquid syringe and handling properties for injection, and in which a chemically crosslinked gel produced by mixing two liquids can exhibit favorable adhesive strength within an appropriate time.

### DESCRIPTION OF EMBODIMENTS

An embodiment of the disclosure will be described below in detail. However, the disclosure is not limited to the following embodiments. In the following embodiments, the constituent elements (including element steps and the like) are not essential unless otherwise specified. The same applies to numerical values and ranges thereof, and the disclosure is not limited thereto.

In the disclosure, a numerical range expressed using "to" includes the numerical values stated before and after "to" as the minimum value and the maximum value, respectively.

In numerical ranges described stepwise in the disclosure, an upper limit value or a lower limit value stated in one numerical range may be replaced with an upper limit value or a lower limit value of another numerical range described stepwise. In a numerical range indicated in the disclosure, an upper limit value or a lower limit value of a numerical range may be replaced with a value shown in the examples.

In the disclosure, each component may include plural kinds of substances corresponding thereto. In a case where plural kinds of substances corresponding to each component are present in a composition, a proportional content or a content of each component means a total content rate or a total content of the plural kinds of substances present in the composition, unless otherwise specified.

### <Adhesive Composition>

This adhesive composition of the disclosure includes a first liquid containing gelatin having an amino group; and a second liquid containing a crosslinking agent having at least two functional groups capable of crosslinking reaction with the amino group in one molecule and a polyethylene glycol skeleton, in which a pH of the first liquid at 25°C is from 6.5 to 9.0, a storage modulus of the first liquid at 25°C is 1.0 Pa or less, an amino group concentration of the first liquid is from 4.0 mM to 40.0 mM, a functional group concentration in terms of the functional group contained in the crosslinking agent in the second liquid is from 2.0 mM to 65.0 mM, and a weight average molecular weight of the crosslinking agent is 10000 or more.

According to the adhesive composition of the disclosure, a chemically crosslinked gel prepared by a crosslinking reaction through two-liquid mixing of the first liquid and the second liquid exhibits favorable adhesive strength. **In** addition, the chemically crosslinked gel has appropriate gel hardness. Furthermore, the adhesive composition of the disclosure can be injected by filling the first liquid and the second liquid into each syringe, and can be solidified within an appropriate time. The adhesive composition of the disclosure is excellent in handleability at room temperature.

Hereinafter, details of each component constituting the adhesive composition of the disclosure will be described. This adhesive composition of the disclosure includes a first liquid containing gelatin having an amino group and a second liquid containing a crosslinking agent (hereinafter, may be referred to as a "specific crosslinking agent") having at least two functional groups capable of crosslinking reaction with the amino group in one molecule and a polyethylene glycol skeleton.

### (First Liquid)

The first liquid according to the disclosure contains gelatin having an amino group.

The gelatin contained in the first liquid is not particularly limited, and examples thereof include natural gelatins derived from mammals such as human, horse, pig, mouse, and rat, natural gelatins derived from fish such as walleye pollock, tilapia, and salmon, and genetically modified gelatins thereof.

The gelatin may be an acid-treated gelatin or an alkali-treated gelatin.

The gelatin may be used singly or two or more kinds thereof may be used in combination. In a case in which two or more kinds of gelatin are used in combination, a proportion of porcine-derived gelatin to a total amount of the gelatin is preferably 50 mass% or more, more preferably 70 mass% or more, and still more preferably 90 mass% or more.

An average molecular weight of the gelatin is not particularly limited.

From the viewpoint of improving the adhesive strength, the gelatin may be subjected to a hydrophobizing treatment. A method of hydrophobizing gelatin is not particularly limited. For example, an acid chloride having a hydrophobic functional group having reactivity with an amino group contained in the gelatin may be mixed with the gelatin dissolved in an organic solvent in the presence of an amine-based catalyst to perform a hydrophobizing treatment.

Examples of the hydrophobic functional group include a cholesteryl group, an oleyl group, an isostearyl group, a stearyl group, an isopalmityl group, a myristyl group, a lauryl group, a caprine group, a pelargonyl group, a caprylyl group, an eicosapentaenoyl group, and a docosahexaenyl group.

The pH of the first liquid at 25°C is from 6.5 to 9.0 from the viewpoint of setting the gelation time of the adhesive composition within an appropriate range. When the pH of the first liquid at 25°C is 6.5 or more, adhesion can be completed in a short time when the adhesive composition of the disclosure is used as an adhesive. In addition, when the pH of the first liquid at 25°C is 9.0 or less, the adhesion time will not be too short, making it suitable for use on a complicated adhesion surface.

The pH of the first liquid at 25°C is preferably from 6.5 to 9.0, more preferably from 7.0 to 8.5, and still more preferably from 7.5 to 8.0 from the viewpoints of use in a physiological environment and improvement in handling properties.

The pH of the first liquid at 25°C refers to a value measured with a glass electrode pH meter (the same applies hereinafter).

The first liquid contains water as a solvent for dissolving gelatin. From the viewpoint of setting the pH of the first liquid at 25°C within a predetermined range and keeping the change in pH constant, the water contained in the first liquid may be a buffer solution having a buffering action with respect to a hydrogen ion concentration. The buffer solution is appropriately selected in view of the pH of the first liquid at 25°C. Examples of the buffer solution include a borate buffer solution, a phosphate buffer solution, an ammonium chloride buffer solution, and a phosphate buffered saline.

The storage modulus of the first liquid at 25°C is 1.0 Pa or less. The storage modulus of the first liquid at 25°C is preferably 0.8 Pa or less, and more preferably 0.5 Pa or less, from the viewpoint of improving handleability at room temperature. The storage modulus of the first liquid at 25°C may be 0 Pa or more.

The storage modulus of the first liquid at 25°C refers to a value measured under an environment at a temperature of 25°C by a rotary viscometer equipped with a cone-plate type measurement jig.

The storage modulus of the first liquid at 25°C can be adjusted to fall within a predetermined range by adjusting the concentration of gelatin contained in the first liquid, the pH of the first liquid, and the like.

From the viewpoints of improving the handleability at room temperature and obtaining favorable adhesive strength, the amino group concentration of the first liquid is from 4.0 mM to 40.0 mM, preferably from 5.0 mM to 35.0 mM, and more preferably from 10.0 mM to 32.0 mM. When the amino group concentration of the first liquid is 4.0 mM or more, sufficient adhesive strength can be secured. When the amino group concentration of the first liquid is 40.0 mM or less, handleability at room temperature is improved.

The amino group concentration of the first liquid can be adjusted to fall within a predetermined range by adjusting the amount of gelatin contained in the first liquid, the amount of amino groups contained in the gelatin, the amount of hydrophobic functional groups introduced into the gelatin, and the like.

The amino group concentration of the first liquid refers to a value measured by a TNBS method described later.

The gelatin concentration contained in the first liquid is preferably from 5 g/L to 100 g/L, more preferably from 10 g/L to 80 g/L, and still more preferably from 20 g/L to 70 g/L.

The first liquid may contain other components in addition to the gelatin and water (buffer solution). Examples of other components contained in the first liquid include colorants, viscosity modifiers, and antibacterial agents.

### (Second Liquid)

The second liquid according to the disclosure contains a specific crosslinking agent. The specific crosslinking agent acts as a crosslinking agent for gelatin.

The functional group capable of crosslinking reaction with the amino group contained in the specific crosslinking agent is not particularly limited, and examples thereof include a carboxy group, an acid chloride, an N-hydroxysuccinimidyl (NHS) group, an aldehyde group, a carbonate group, a carbodiimide group, an isothiocyanate group, a sulfosuccinimidyl group, and a phthalimidyl group. Among them, an NHS group is preferable from the viewpoint of being capable of reacting with an amino group contained in gelatin under a physiological condition to a slightly alkaline condition (pH (25°C): from 7.2 to 9.0) to form a stable amide bond.

The functional group concentration in terms of the functional group capable of crosslinking reaction with the amino group contained in the specific crosslinking agent in the second liquid is from 2.0 mM to 65.0 mM, preferably from 3.0 mM to 55.0 mM, and more preferably from 4.0 mM to 45.0 mM, from the viewpoint of obtaining sufficient adhesive strength. When the functional group concentration in the second liquid is 2.0 mM or more, sufficient adhesive strength can be secured. It is preferable that the functional group concentration in the second liquid be 65.0 mM or less, from the viewpoint of developing the adhesive strength of the chemically crosslinked gel.

The functional group concentration in terms of the functional group capable of crosslinking reaction with the amino group contained in the specific crosslinking agent in the second liquid can be adjusted to fall within a predetermined range by adjusting the concentration of the specific crosslinking agent contained in the second liquid, the amount of the functional groups contained in the specific crosslinking agent, and the like.

A method of measuring the functional group concentration in terms of the functional group capable of crosslinking reaction with the amino group contained in the specific crosslinking agent in the second liquid is appropriately selected according to the kind of the functional group contained in the specific crosslinking agent. For example, in a case in which the functional group is an NHS group, the functional group concentration in the second liquid refers to a value calculated based on the molecular weight, the number of functional groups, and the concentration of the specific crosslinking agent in the second liquid.

A ratio (amino group concentration/functional group concentration) of the amino group concentration of the first liquid to the functional group concentration in terms of the functional group capable of crosslinking reaction with the amino group contained in the specific crosslinking agent in the second liquid is preferably from 0.2 to 7.0, more preferably from 0.3 to 7.0, and still more preferably from 0.35 to 6.0 from the viewpoint of securing sufficient adhesive strength.

The concentration of the specific crosslinking agent in the second liquid is preferably 10 g/L or more, and more preferably 30 g/L from the viewpoint of obtaining sufficient adhesive strength. The concentration of the specific crosslinking agent in the second liquid may be 300 g/L or less from the viewpoint of exhibiting favorable adhesive strength of the chemically crosslinked gel. The concentration of the specific crosslinking agent in the second liquid is preferably from 10 g/L to 300 g/L.

The pH of the second liquid at 25°C is preferably from 3.0 to 8.0 and more preferably from 4.0 to 6.0 from the viewpoint of controlling the pH when the first liquid and the second liquid are mixed.

In addition, the difference between the pH of the first liquid at 25°C and the pH of the second liquid at 25°C is preferably from 0 to 6.0 and more preferably from 0 to 4.0 from the viewpoint of controlling the pH when the first liquid and the second liquid are mixed to a range close to the pH of the first liquid.

The pH of the second liquid at 25°C refers to a value measured by the same method as the method of measuring the pH of the first liquid.

The weight average molecular weight (Mw) of the specific crosslinking agent is 10000 or more, and preferably 20000 or more, from the viewpoint of obtaining favorable adhesive strength. The weight average molecular weight of the specific crosslinking agent may be 40000 or less from the viewpoint of securing the strength of the chemically crosslinked gel. The weight average molecular weight of the specific crosslinking agent is preferably from 10000 to 40000.

The weight average molecular weight of the specific crosslinking agent refers to a value measured by gel permeation chromatography.

The specific crosslinking agent may be a compound represented by the following Formula (A).

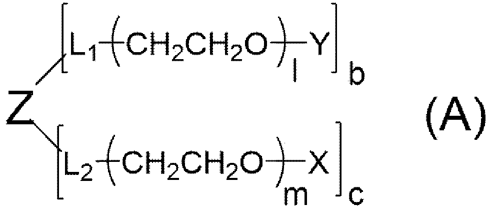

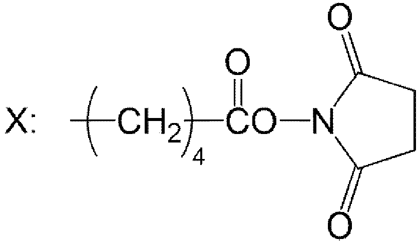

In Formula (A), Z represents a (b+c)-valent organic group, Y represents a hydrogen atom or a monovalent organic group, L₁ and L₂ each independently represent -O- or -O-CONH-(CH₂)₃-O-, b represents an integer from 0 to 5, c represents an integer from 3 to 8, (b+c) represents an integer from 3 to 8, l represents 0 or an integer of 1 or more, and m represents an integer of 1 or more.

The (b+c)-valent organic group represented by Z in Formula (A) is not particularly limited. The (b+c)-valent organic group represented by Z may be composed of a carbon atom and a hydrogen atom, or may further contain an oxygen atom, a sulfur atom, a nitrogen atom, or the like. In a case in which the (b+c)-valent organic group represented by Z contains an oxygen atom, the (b+c)-valent organic group represented by Z may contain an ether bond in the structure.

Specific examples of the (b+c)-valent organic group represented by Z include organic groups represented by the following Structural Formulas (C) to (J). In the following structural formulas, * represents a bond to L₁ or L₂.

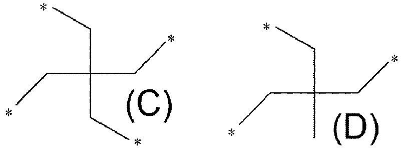

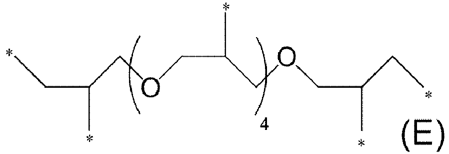

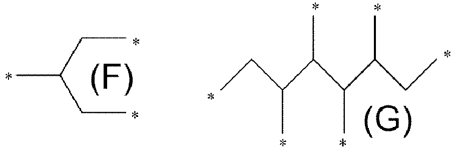

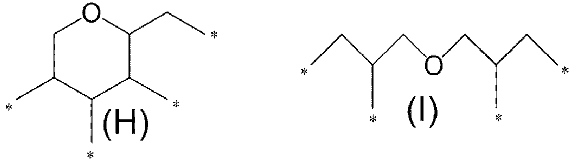

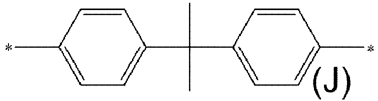

In Formula (A), examples of the monovalent organic group represented by Y include an alkyl group, an aryl group, and an acyl group. In Formula (A), Y is preferably a hydrogen atom or an alkyl group.

In Formula (A), L₁ and L₂ are preferably -O-.

In Formula (A), b is preferably from 0 to 3, more preferably 0 or 1, and still more preferably 0.

In Formula (A), c is preferably from 4 to 8.

In Formula (A), (b+c) is preferably from 4 to 8.

In Formula (A), l and m are appropriately set so that the weight average molecular weight of the specific crosslinking agent falls within the preferable range. In a case in which b and c are 2 or more, a plurality of l and m may have the same numerical value or different numerical values.

The specific crosslinking agent may be a compound represented by the following Formula (B).

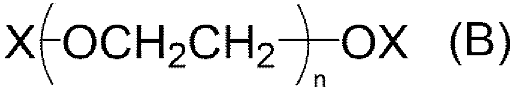

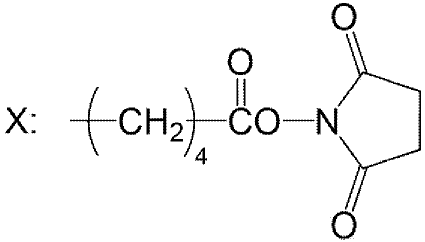

In Formula (B), n represents an integer of 1 or more. In Formula (B), n is appropriately set so that the weight average molecular weight of the specific crosslinking agent falls within the preferable range.

As the specific crosslinking agent, the compound represented by Formula (A) and the compound represented by Formula (B) may be used in combination.

In the second liquid, a crosslinking agent other than the specific crosslinking agent may be used in combination. Examples of other crosslinking agents include genipin, tartaric acid, citric acid, malic acid, glutamic acid, aspartic acid, oxaloacetic acid, aconitic acid, 2-ketoglutaric acid, polytartaric acid, polycitric acid, polymalic acid, polyglutamic acid, and polyaspartic acid.

In a case in which the second liquid contains another crosslinking agent, a proportion of the specific crosslinking agent to a total of the crosslinking agents contained in the second liquid is preferably 50 mass% or more, more preferably 70 mass% or more, and still more preferably 90 mass% or more.

The second liquid contains water as a solvent for dissolving the specific crosslinking agent. From the viewpoint of setting the pH of the second liquid at 25°C within a predetermined range and keeping the change in pH constant, the water contained in the second liquid may be a buffer solution having a buffering action with respect to a hydrogen ion concentration. The buffer solution is appropriately selected in view of the pH of the second liquid at 25°C. Examples of the buffer solution include a borate buffer solution, a phosphate buffer solution, an ammonium chloride buffer solution, and a phosphate buffered saline.

The second liquid may contain other components in addition to the crosslinking agent and water (buffer solution). Examples of other components contained in the second liquid include colorants, viscosity modifiers, and antibacterial agents.

### <Method of Producing Adhesive Composition>

The method for producing the adhesive composition of the disclosure is not particularly limited, and is appropriately selected according to kind of the gelatin and specific crosslinking agent to be used.

The first liquid can be prepared, for example, by dissolving gelatin in a buffer solution. The buffer solution may be heated when the gelatin is dissolved in the buffer solution. A temperature of the buffer solution is preferably from 25°C to 50°C. The prepared first liquid may simply be filled into a container separate from the second liquid. The prepared first liquid may be filtered by using a filter such as a mesh filter, a membrane filter, a nonwoven filter, or a sintered filter.

The second liquid can be prepared, for example, by dissolving a specific crosslinking agent in a buffer solution. The buffer solution may be heated, when the specific crosslinking agent is dissolved in the buffer solution. A temperature of the buffer solution is preferably from 20°C to 30°C. The prepared second liquid may be filtered in the same manner as the first liquid. The prepared second liquid may simply be filled into a container separate from the first liquid.

### <Method of Using Adhesive Composition>

The adhesive composition of the disclosure exhibits a function as an adhesive by mixing the first liquid and the second liquid. When mixing the first liquid and the second liquid, a crosslinking reaction occurs between the amino group of the gelatin contained in the first liquid and the functional group of the specific crosslinking agent contained in the second liquid, and a chemically strong bond is formed. This makes it possible to obtain high adhesive strength.

A mixture of the first liquid and the second liquid is applied to the tissue to be adhered and is left for a predetermined time, whereby the applied mixture solidifies. According to this, the tissue is adhered.

A reaction rate of the crosslinking reaction can be adjusted by the pH of the first liquid and the second liquid. Therefore, when the solidification rate is appropriately set by adjusting the pH of the first liquid and the second liquid, the adhesive composition of the disclosure can be easily applied to a complicated adhesion surface.

A mixing ratio of the first liquid and the second liquid is not particularly limited. The mixing ratio of the first liquid and the second liquid may be adjusted so that the pH when the first liquid and the second liquid are mixed becomes a value close to the pH of the first liquid.

A method of mixing the first liquid and the second liquid is not particularly limited. For example, the first liquid and the second liquid can be mixed using a two-liquid mixing device such as a two-liquid mixing syringe, a two-component dispenser, or an air-assisted spray including a two-liquid syringe.

### [Examples]

Hereinafter, the disclosure will be described more specifically with reference to examples, but the disclosure is not limited to the following examples, as long as it does not depart from the gist of the disclosure.

### (Structure of Crosslinking Agent)

Chemical structures of specific crosslinking agents used in Examples and Comparative Examples are as follows. A weight average molecular weight Mw is from 10000 to 40000, and each n in the formula is independently from 57 to 228, approximately. With respect to the crosslinking agent used in each of Examples and Comparative Examples, the number of functional groups, Mw, crosslinking agent concentration in the second liquid, and functional group concentration in terms of the functional groups contained in the crosslinking agent in the second liquid are as shown in Table 2.

The functional group concentration was determined by calculation based on, for example, the number of functional groups contained in the crosslinking agent, the Mw of the crosslinking agent, and the crosslinking agent concentration.

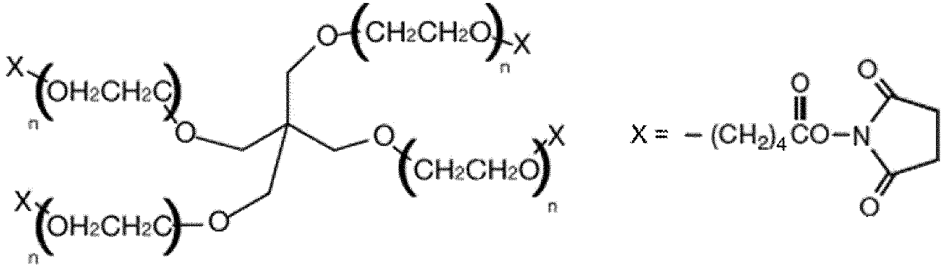

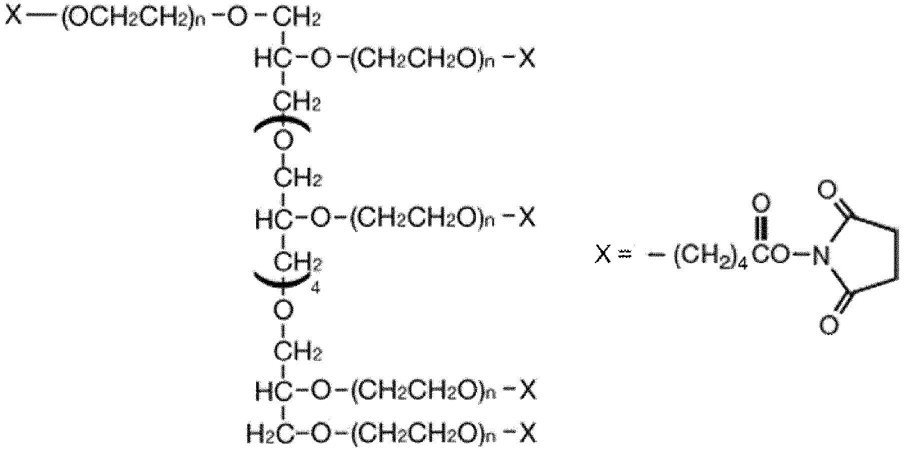

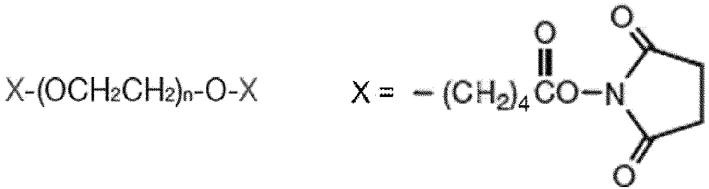

### (Structure of Gelatin)

Acid-treated porcine skin gelatin (manufactured by Nitta Gelatin Inc.) was used as gelatin. As shown in Table 1 below, in the amino sequence of the acid-treated porcine skin gelatin, 27 lysine residues (Lys) are contained as amino acids having amino groups per 1000 residues. The molecular weight distribution of the acid-treated porcine skin gelatin used is tens of thousands to millions.

**[Table 1]**

| Amino acid | Abbreviation | Amino acid composition (per 1000 residues) |
|---|---|---|
| Glycine | Gly | 330 |
| Alanine | Ala | 112 |
| Valine | Val | 26 |
| Leucine | Leu | 24 |
| Isoleucine | Ile | 10 |
| Serine | Ser | 35 |
| Threonine | Thr | 18 |
| Aspartic acid | Asp | 45 |
| Glutamic acid | Glu | 72 |
| Cystine | Cys | 0 |
| Methionine | Met | 4 |
| Lysine | Lys | 27 |
| Hydroxylysine | Hyl | 6 |
| Arginine | Arg | 49 |
| Histidine | His | 4 |
| Phenylalanine | Phe | 13 |
| Tyrosine | Tyr | 3 |
| Tryptophan | Trp | 0 |
| Proline | Pro | 131 |
| Oxyproline | Hyp | 91 |

### <Preparation of Acid-Treated Porcine Skin Gelatin Aqueous Solution (First Liquid) And Multibranched PEG Aqueous Solution (Second Liquid), And Production of Chemically Crosslinked Gel>

A 0.1 M borate buffer solution and a 0.01 M phosphate buffer solution were prepared and used for the preparation of the first liquid and the second liquid. A 2 M aqueous hydrochloric acid solution or a 2 M aqueous sodium hydroxide solution was added to adjust the pH of the buffer solution. The pH of the buffer solution at 25°C was measured with a benchtop pH meter F-72 manufactured by HORIBA, Ltd.

### <Example 1>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 2>

Acid-treated porcine skin gelatin (BM-PS1052, manufactured by Nitta Gelatin Inc.) was dissolved in a 0.1 M borate buffer solution (pH 9.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 3>

Acid-treated porcine skin gelatin (BM-PS1052, manufactured by Nitta Gelatin Inc.) was dissolved in a 0.1 M borate buffer solution (pH 8.5) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 4>

Acid-treated porcine skin gelatin (BM-PS1052, manufactured by Nitta Gelatin Inc.) was dissolved in a 0.1 M borate buffer solution (pH 7.5) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 5>

Acid-treated porcine skin gelatin (BM-PS1052, manufactured by Nitta Gelatin Inc.) was dissolved in a 0.1 M borate buffer solution (pH 7.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 6>

Acid-treated porcine skin gelatin (BM-PS1053, manufactured by Nitta Gelatin Inc.) was dissolved in a 0.1 M borate buffer solution (pH 8.0) in an amount to give a gelatin concentration of 23 g/L to obtain an acid-treated porcine skin gelatin aqueous solution (first liquid). When an amino group concentration of the acid-treated porcine skin gelatin aqueous solution was quantified by a TNBS method described later, it was 7.9 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 7>

Acid-treated porcine skin gelatin (BM-PS1051, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 21.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 8>

Acid-treated porcine skin gelatin (BM-PS1051, manufactured by Nitta Gelatin Inc.) was dissolved in a 0.1 M borate buffer solution (pH 8.0) in an amount to give a gelatin concentration of 69 g/L to obtain an acid-treated porcine skin gelatin aqueous solution (first liquid). When an amino group concentration of the acid-treated porcine skin gelatin aqueous solution was quantified by a TNBS method described later, it was 31.7 mM.

Separately, the specific crosslinking agent (SUNBRIGHT PTE-200HS, NOF CORPORATION) was dissolved in a 0.01 M phosphate buffer solution (pH 4.0) in an amount to be 99 g/L to obtain a crosslinking agent aqueous solution (second liquid). A functional group concentration of the crosslinking agent aqueous solution was 19.8 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 9>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 16 g/L to obtain a crosslinking agent aqueous solution (second liquid). A functional group concentration of the crosslinking agent aqueous solution was 3.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 10>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 99 g/L to obtain a crosslinking agent aqueous solution (second liquid). A functional group concentration of the crosslinking agent aqueous solution was 19.8 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 11>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 132 g/L to obtain a crosslinking agent aqueous solution (second liquid). A functional group concentration of the crosslinking agent aqueous solution was 26.4 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 12>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 198 g/L to obtain a crosslinking agent aqueous solution (second liquid). A functional group concentration of the crosslinking agent aqueous solution was 39.6 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 13>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT HGEO-200GS, NOF CORPORATION) was dissolved in a 0.01 M phosphate buffer solution (pH 4.0) in an amount to be 33 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 14>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT HGEO-200GS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 26.4 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 15>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT DE-200HS, NOF CORPORATION) was dissolved in a 0.01 M phosphate buffer solution (pH 4.0) in an amount to be 132 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 16>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT DE-200HS, NOF CORPORATION) was dissolved in a 0.01 M phosphate buffer solution (pH 4.0) in an amount to be 264 g/L to obtain a crosslinking agent aqueous solution (second liquid). A functional group concentration of the crosslinking agent aqueous solution was 26.4 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 17>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-100HS, NOF CORPORATION) was dissolved in a 0.01 M phosphate buffer solution (pH 4.0) in an amount to be 33 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 18>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-100HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 26.4 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 19>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-400HS, NOF CORPORATION) was dissolved in a 0.01 M phosphate buffer solution (pH 4.0) in an amount to be 132 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 20>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-400HS, NOF CORPORATION) was dissolved in a 0.01 M phosphate buffer solution (pH 4.0) in an amount to be 264 g/L to obtain a crosslinking agent aqueous solution (second liquid). A functional group concentration of the crosslinking agent aqueous solution was 26.4 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Example 21>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 300 g/L to obtain a crosslinking agent aqueous solution (second liquid). A functional group concentration of the crosslinking agent aqueous solution was 60.0 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel.

### <Comparative Example 1>

Acid-treated porcine skin gelatin (BM-PS1052, manufactured by Nitta Gelatin Inc.) was dissolved in a 0.1 M borate buffer solution (pH 8.0) in an amount to give a gelatin concentration of 92 g/L to obtain an acid-treated porcine skin gelatin aqueous solution (first liquid). When an amino group concentration of the acid-treated porcine skin gelatin aqueous solution was quantified by a TNBS method described later, it was 32.2 mM. The gelatin solidified at 25°C and thus could not be filled into a syringe. Therefore, the evaluation was stopped at this stage.

### <Comparative Example 2>

Acid-treated porcine skin gelatin (BM-PS1051, manufactured by Nitta Gelatin Inc.) was dissolved in a 0.1 M borate buffer solution (pH 8.0) in an amount to give a gelatin concentration of 92 g/L to obtain an acid-treated porcine skin gelatin aqueous solution (first liquid). When an amino group concentration of the acid-treated porcine skin gelatin aqueous solution was quantified by a TNBS method described later, it was 42.2 mM. The gelatin solidified at 25°C and thus could not be filled into a syringe. Therefore, the evaluation was stopped at this stage.

### <Comparative Example 3>

Acid-treated porcine skin gelatin (BM-PS1052, manufactured by Nitta Gelatin Inc.) was dissolved in a 0.1 M borate buffer solution (pH 8.0) in an amount to give a gelatin concentration of 184 g/L to obtain an acid-treated porcine skin gelatin aqueous solution (first liquid). When an amino group concentration of the acid-treated porcine skin gelatin aqueous solution was quantified by a TNBS method described later, it was 64.4 mM. The gelatin solidified at 25°C and thus could not be filled into a syringe. Therefore, the evaluation was stopped at this stage.

### <Comparative Example 4>

Acid-treated porcine skin gelatin (BM-PS1053, manufactured by Nitta Gelatin Inc.) was dissolved in a 0.1 M borate buffer solution (pH 8.0) in an amount to give a gelatin concentration of 12 g/L to obtain an acid-treated porcine skin gelatin aqueous solution (first liquid). When an amino group concentration of the acid-treated porcine skin gelatin aqueous solution was quantified by a TNBS method described later, it was 3.8 mM.

Separately, the specific crosslinking agent (SUNBRIGHT PTE-200HS, NOF CORPORATION) was dissolved in a 0.01 M phosphate buffer solution (pH 4.0) in an amount to be 24 g/L to obtain a crosslinking agent aqueous solution (second liquid). A functional group concentration of the crosslinking agent aqueous solution was 4.8 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel. A storage modulus of the resulting chemically crosslinked gel was insufficient.

### <Comparative Example 5>

Acid-treated porcine skin gelatin (BM-PS1053, manufactured by Nitta Gelatin Inc.) was dissolved in a 0.1 M borate buffer solution (pH 8.0) in an amount to give a gelatin concentration of 12 g/L to obtain an acid-treated porcine skin gelatin aqueous solution (first liquid). When an amino group concentration of the acid-treated porcine skin gelatin aqueous solution was quantified by a TNBS method described later, it was 3.8 mM.

Separately, the specific crosslinking agent (SUNBRIGHT PTE-200HS, NOF CORPORATION) was dissolved in a 0.01 M phosphate buffer solution (pH 4.0) in an amount to be 33 g/L to obtain a crosslinking agent aqueous solution (second liquid). A functional group concentration of the crosslinking agent aqueous solution was 6.6 mM.

The acid-treated porcine skin gelatin aqueous solution and the crosslinking agent aqueous solution were mixed in equal volumes under an environment of 25°C, and a crosslinking reaction and three-dimensional network formation were performed to obtain a chemically crosslinked gel. A storage modulus of the resulting chemically crosslinked gel was insufficient.

### <Comparative Example 6>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 8 g/L to obtain a crosslinking agent aqueous solution (second liquid). A functional group concentration of the crosslinking agent aqueous solution was 1.6 mM.

Acid-treated porcine skin gelatin and a crosslinking agent aqueous solution were mixed in equal volumes under an environment of 25°C, but a chemically crosslinked gel was not obtained.

### <Comparative Example 7>

Acid-treated porcine skin gelatin (BM-PS1052, manufactured by Nitta Gelatin Inc.) was dissolved in a 0.1 M borate buffer solution (pH 6.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, but a chemically crosslinked gel was not obtained within 600 seconds.

### <Comparative Example 8>

Acid-treated porcine skin gelatin (BM-PS1052, manufactured by Nitta Gelatin Inc.) was dissolved in a 0.1 M borate buffer solution (pH 9.5) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 66 g/L to obtain a crosslinking agent aqueous solution (second liquid). A functional group concentration of the crosslinking agent aqueous solution was 13.2 mM.

The acid-treated porcine skin gelatin aqueous solution and the crosslinking agent aqueous solution were mixed in equal volumes under an environment of 25°C, but since the formation of the chemically crosslinked gel was too rapid, the chemically crosslinked gel clogged at a tip portion of a syringe during the extrusion from the two-liquid syringe.

### <Comparative Example 9>

Acid-treated porcine skin gelatin (BM-PS1053, manufactured by Nitta Gelatin Inc.) was dissolved in a 0.1 M borate buffer solution (pH 8.0) in an amount to give a gelatin concentration of 80 g/L to obtain an acid-treated porcine skin gelatin aqueous solution (first liquid). When an amino group concentration of the acid-treated porcine skin gelatin aqueous solution was quantified by a TNBS method described later, it was 28.0 mM. The gelatin solidified at 25°C and thus could not be filled into a syringe. Therefore, the evaluation was stopped at this stage.

### <Comparative Example 10>

Acid-treated porcine skin gelatin (BM-PS1052, manufactured by Nitta Gelatin Inc.) was dissolved in a 0.1 M borate buffer solution (pH 8.0) in an amount to give a gelatin concentration of 46 g/L to obtain an acid-treated porcine skin gelatin aqueous solution (first liquid). When an amino group concentration of the acid-treated porcine skin gelatin aqueous solution was quantified by a TNBS method described later, it was 16.1 mM.

Separately, the specific crosslinking agent (SUNBRIGHT PTE-200HS, NOF CORPORATION) was dissolved in a 0.01 M phosphate buffer solution (pH 4.0) in an amount to be 350 g/L to obtain a crosslinking agent aqueous solution (second liquid). A functional group concentration of the crosslinking agent aqueous solution was 70.0 mM.

The acid-treated porcine skin gelatin aqueous solution and the crosslinking agent aqueous solution were mixed in equal volumes under an environment of 25°C, but a chemically crosslinked gel was not obtained.

### <Method of Quantifying Amino Group Concentration>

The amino group concentration derived from the lysine residues of the acid-treated porcine skin gelatin aqueous solution was quantified by the following sodium trinitrobenzenesulfonate (TNBS) method.

49 mg of sodium 2,4,6-trinitrobenzenesulfonate dihydrate was weighed precisely, and dissolved in distilled water to make exactly 1 mL. This was used as a TNBS solution. 50 mg of acid-treated porcine skin gelatin was weighed precisely, and dissolved in distilled water to make exactly 10 mL of the solution, which was used as a sample solution. A solution in which 45 mg of phenylalanine was precisely weighed and dissolved in distilled water to make exactly 1 mL was prepared. Distilled water was added to each of 100 µL, 250 µL, 500 µL, and 750 µL of the left-described phenylalanine solution to make exactly 1 mL, and these solutions were used as standard solutions.

Next, 100 µL each of distilled water, a sample solution, and each standard solution was measured into test tubes, and 3 mL of a 0.1 M sodium borate solution and 30 µL of a 0.2 M sodium sulfite aqueous solution were added to each test tube. Further, 100 µL of the TNBS solution was added to each test tube, stirred, mixed, and allowed to stand for 40 minutes.

Thereafter, the absorbance at a wavelength of 420 nm was measured with a spectrophotometer, and a value obtained by subtracting the absorbance of the control operated using distilled water from the obtained absorbance was taken as an absorbance of the sample solution. A calibration curve was created with the absorbance on the vertical axis and the amino group concentration derived from the chemical structure of phenylalanine on the horizontal axis, and the amino group concentration of the sample solution was determined from the intersection of the calibration curve and the absorbance of the sample solution.

### <<Evaluation Test>>

The following test was performed on the chemically crosslinked gel. Each evaluation test was performed in a thermostatic chamber at a temperature of 25°C. The evaluation results are shown in Table 2.

### <Evaluation Test 1>: Viscoelasticity Evaluation And Fluidity Evaluation of Gelatin

With respect to the acid-treated porcine skin gelatin aqueous solution (2 mL), the storage modulus (G') at a frequency of 1 Hz and a strain of 0.1% at 25°C was measured with a rheometer (MCR302 (Anton Paar)) using a cone-plate type measuring jig.

In addition, a vial containing the acid-treated porcine skin gelatin aqueous solution was inclined, and the presence or absence of fluidity was confirmed.
A: Fluidity was confirmed.
C: No fluidity was confirmed.

### <Evaluation Test 2>: Measurement of Gelation Time

The acid-treated porcine skin gelatin aqueous solution and the crosslinking agent aqueous solution were mixed in equal volumes, and a crosslinking reaction between the gelatin and the crosslinking agent and three-dimensional network formation were performed. In Examples, a clear chemically crosslinked gel was formed. The time from the time point when the acid-treated porcine skin gelatin aqueous solution and the crosslinking agent aqueous solution were mixed to the time point when the vial containing the mixed solution was inclined and no fluidity was confirmed was taken as the gelation time. The gelation time was evaluated according to the following evaluation criteria.

### (Evaluation Criteria of Gelation Time)

A: 30 seconds or more but less than 300 seconds.
B: 10 seconds or more but less than 30 seconds, or 300 seconds or more but less than 600 seconds.
C: Less than 10 seconds, or 600 seconds or more.

### <Evaluation Test 3>: Viscoelasticity Evaluation of Chemically Crosslinked Gel

With respect to the chemically crosslinked gel (2 mL), the storage modulus (G') at a temperature of 25°C, a frequency of 1 Hz, and a strain of 0.1% was measured with a rheometer (MCR302 (Anton Paar)) using a cone-plate type measurement jig. A graph was created with the storage modulus (G') on the vertical axis and the time from two-liquid mixing on the horizontal axis, and the storage modulus at 10 minutes after mixing was read. G' was evaluated according to the following evaluation criteria.

### (Evaluation Criteria for Storage Modulus of Chemically Crosslinked Gel)

A: 100 Pa or more.
B: 10 Pa or more but less than 100 Pa.
C: Less than 10 Pa.

### <Evaluation Test 4>: Adhesion Test of Chemically Crosslinked Gel

A collagen casing (manufactured by Nippi Co., Ltd.) was fixed with a masking tape on a base formed by superimposing slide glass test pieces. 25 µL of an acid-treated porcine skin gelatin aqueous solution was added dropwise to one side of an adhesion surface (0.5 cm × 2.5 cm), and 25 µL of a crosslinking agent aqueous solution was added dropwise to the other side. The surfaces were bonded to each other, both ends were fixed with clips, and were allowed to stand for 10 minutes at room temperature around 25°C. Thereafter, a vertical tensile test was performed using TENSILON ORIENTEC RTF-1210 (product name) manufactured by A & D Company, Limited, at room temperature around 25°C and a tensile speed of 30 mm/min.

### (Evaluation Criteria of Adhesive Strength)

A: 0.2 N/cm² or more.
B: 0.1 N/cm² or more but less than 0.2 N/cm².
C: Less than 0.1 N/cm².

**[Table 2]**

| | Gelatin | | | | Crosslinking agent | | | | | Gelatin | Chemically crosslinked gel | | | | | |
|---|---|---|---|---|---|---|---|---|---|---|---|---|---|---|---|---|
| | pH | G' (Pa) (25°C) | Amino group concentration (mM) | Concentration (g/L) | pH | Number of functional groups | Mw × 10⁴ | Concentration (g/L) | Functional group concentration (mM) | Fluidity | Gelation time (s) | Gelation time evaluation | Storage modulus (Pa) | Storage modulus evaluation | Adhesive strength (N/cm²) | Adhesive strength evaluation |
| Example 1 | 8.0 | 0.3 | 16.1 | 46 | 4.0 | 4 | 2 | 66 | 13.2 | A | 41 | A | 740 | A | 1.3 | A |
| Example 2 | 9.0 | 0.3 | 16.1 | 46 | 4.0 | 4 | 2 | 66 | 13.2 | A | 14 | B | 640 | A | 1.0 | A |
| Example 3 | 8.5 | 0.2 | 16.1 | 46 | 4.0 | 4 | 2 | 66 | 13.2 | A | 20 | B | 1052 | A | 1.3 | A |
| Example 4 | 7.5 | 0.3 | 16.1 | 46 | 4.0 | 4 | 2 | 66 | 13.2 | A | 124 | A | 2370 | A | 1.5 | A |
| Example 5 | 7.0 | 0.3 | 16.1 | 46 | 4.0 | 4 | 2 | 66 | 13.2 | A | 390 | B | 34 | B | 0.1 | B |
| Example 6 | 8.0 | 0.3 | 7.9 | 23 | 4.0 | 4 | 2 | 66 | 13.2 | A | 102 | A | 920 | A | 0.7 | A |
| Example 7 | 8.0 | 0.1 | 21.1 | 46 | 4.0 | 4 | 2 | 66 | 13.2 | A | 102 | A | 520 | A | 0.7 | A |
| Example 8 | 8.0 | 0.4 | 31.7 | 69 | 4.0 | 4 | 2 | 99 | 19.8 | A | 46 | A | 714 | A | 1.2 | A |
| Example 9 | 8.0 | 0.3 | 16.1 | 46 | 4.0 | 4 | 2 | 16 | 3.2 | A | 220 | A | 26 | B | 0.1 | B |
| Example 10 | 8.0 | 0.3 | 16.1 | 46 | 4.0 | 4 | 2 | 99 | 19.8 | A | 40 | A | 680 | A | 0.9 | A |
| Example 11 | 8.0 | 0.3 | 16.1 | 46 | 4.0 | 4 | 2 | 132 | 26.4 | A | 31 | A | 1020 | A | 1.3 | A |
| Example 12 | 8.0 | 0.3 | 16.1 | 46 | 4.0 | 4 | 2 | 198 | 39.6 | A | 92 | A | 2120 | A | 1.5 | A |
| Example 13 | 8.0 | 0.3 | 16.1 | 46 | 4.0 | 8 | 2 | 33 | 13.2 | A | 52 | A | 750 | A | 1.3 | A |
| Example 14 | 8.0 | 0.3 | 16.1 | 46 | 4.0 | 8 | 2 | 66 | 26.4 | A | 50 | A | 390 | A | 1.1 | A |
| Example 15 | 8.0 | 0.3 | 16.1 | 46 | 4.0 | 2 | 2 | 132 | 13.2 | A | 100 | A | 1100 | A | 0.6 | A |
| Example 16 | 8.0 | 0.3 | 16.1 | 46 | 4.0 | 2 | 2 | 264 | 26.4 | A | 86 | A | 440 | A | 0.5 | A |
| Example 17 | 8.0 | 0.3 | 16.1 | 46 | 4.0 | 4 | 1 | 33 | 13.2 | A | 82 | A | 22 | B | 0.5 | A |
| Example 18 | 8.0 | 0.3 | 16.1 | 46 | 4.0 | 4 | 1 | 66 | 26.4 | A | 61 | A | 31 | B | 0.5 | A |
| Example 19 | 8.0 | 0.3 | 16.1 | 46 | 4.0 | 4 | 4 | 132 | 13.2 | A | 113 | A | 430 | A | 0.7 | A |
| Example 20 | 8.0 | 0.3 | 16.1 | 46 | 4.0 | 4 | 4 | 264 | 26.4 | A | 69 | A | 1980 | A | 1.0 | A |
| Example 21 | 8.0 | 0.3 | 16.1 | 46 | 4.0 | 4 | 2 | 300 | 60.0 | A | 120 | A | 170 | A | 0.2 | A |
| Comparative Example 1 | 8.0 | 15 | 32.2 | 92 | - | - | - | - | - | C | - | - | - | - | - | - |
| Comparative | 8.0 | 4 | 42.2 | 92 | - | - | - | - | - | C | - | - | - | - | - | - |
| Example 2 | | | | | | | | | | | | | | | | |
| Comparative Example 3 | 8.0 | 22 | 64.4 | 184 | - | - | - | - | - | C | - | - | - | - | - | - |
| Comparative Example 4 | 8.0 | 0.4 | 3.8 | 12 | 4.0 | 4 | 2 | 24 | 4.8 | A | 240 | A | 0.5 | C | 0.6 | A |
| Comparative Example 5 | 8.0 | 0.4 | 3.8 | 12 | 4.0 | 4 | 2 | 33 | 6.6 | A | 269 | A | 0.9 | C | 0.7 | A |
| Comparative Example 6 | 8.0 | 0.3 | 16.1 | 46 | 4.0 | 4 | 2 | 8 | 1.6 | A | - | C | - | C | - | C |
| Comparative Example 7 | 6.0 | 0.3 | 16.1 | 46 | 4.0 | 4 | 2 | 66 | 13.2 | A | 1320 | C | 0.4 | C | 0 | C |
| Comparative Example 8 | 9.5 | 0.3 | 16.1 | 46 | 4.0 | 4 | 2 | 66 | 13.2 | A | 9 | C | 180 | A | 1.7 | A |
| Comparative Example 9 | 8.0 | 2 | 28.0 | 80 | - | - | - | - | - | C | - | - | - | - | - | - |
| Comparative Example 10 | 8.0 | 0.3 | 16.1 | 46 | 4.0 | 4 | 2 | 350 | 70.0 | A | - | C | 0.2 | C | 0 | C |

From the results shown in Table 2, it is found that by changing the amino group concentration of the gelatin, the storage modulus, or the concentration of the specific crosslinking agent, the number of functional groups constituting the specific crosslinking agent, and the weight average molecular weight, the gelatin and the specific crosslinking agent can be filled into a two-liquid syringe and injected, and furthermore, a chemically crosslinked gel can be prepared in an appropriate time through two-liquid mixing, and favorable adhesiveness can be exhibited.

The entire disclosure of Japanese Patent Application No. 2023-073852 filed on April 27, 2023 is incorporated herein by reference.

All the literature, patent application, and technical standards cited herein are herein incorporated to the same extent as provided for specifically and severally with respect to an individual literature, patent application, and technical standard to the effect that the same should be so incorporated by reference.

## Claims

1. An adhesive composition comprising:
a first liquid containing gelatin having an amino group; and
a second liquid containing a crosslinking agent having at least two functional groups capable of crosslinking reaction with the amino group in one molecule and a polyethylene glycol skeleton,
wherein:
a pH of the first liquid at 25°C is from 6.5 to 9.0,
a storage modulus of the first liquid at 25°C is 1.0 Pa or less,
an amino group concentration of the first liquid is from 4.0 mM to 40.0 mM,
a functional group concentration in terms of the functional groups contained in the crosslinking agent in the second liquid is from 2.0 mM to 65.0 mM, and
a weight average molecular weight of the crosslinking agent is 10,000 or more.

2. The adhesive composition according to claim 1, wherein the functional groups include an N-hydroxysuccinimidyl (NHS) group.

3. The adhesive composition according to claim 1, wherein the crosslinking agent includes a compound represented by the following Formula (A): 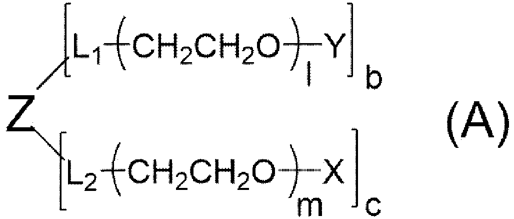 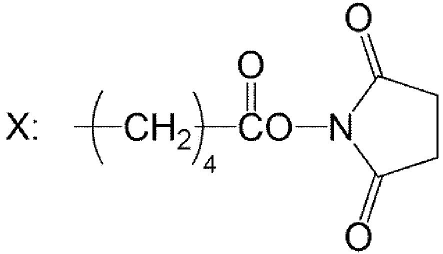 wherein, in Formula (A), Z represents a (b+c)-valent organic group, Y represents a hydrogen atom or a monovalent organic group, L₁ and L₂ each independently represent -O- or -O-CONH-(CH₂)₃-O-, b represents an integer from 0 to 5, c represents an integer from 3 to 8, (b+c) represents an integer from 3 to 8, l represents 0 or an integer of 1 or more, and m represents an integer of 1 or more.

4. The adhesive composition according to claim 1, wherein the crosslinking agent includes a compound represented by the following Formula (B): 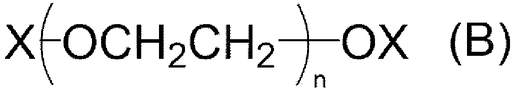 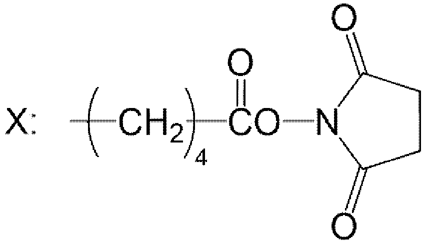 wherein, in Formula (B), n represents an integer of 1 or more.

5. The adhesive composition according to claim 1, wherein a pH of the second liquid at 25°C is from 3.0 to 8.0.

6. The adhesive composition according to claim 1, wherein a difference between the pH of the first liquid at 25°C and a pH of the second liquid at 25°C is from 0 to 6.0.
